# FASCICULE DE BREVET EUROPEEN

(11) **EP 1 722 757 B1**
(45) Date de publication et mention de la délivrance du brevet: **20.05.2009**
(21) Numéro de dépôt: 05736611.4
(22) Date de dépôt: 08.03.2005
(51) Int. Cl.: A61K 8/97, A61Q 19/06

(54) **COMPOSITION COSMETIQUE AMINCISSANTE COMPRENANT EN TANT QU'AGENT ACTIF UN INHIBITEUR DE METALLOPROTEINASES**
KOSMETISCHE ZUSAMMENSETZUNG ZUM ABNEHMEN MIT EINEM METALLOPROTEINASE-HEMMER ALS WIRKSTOFF
SLIMMING COSMETIC COMPOSITION COMPRISING A METALLOPROTEINASE INHIBITOR AS AN ACTIVE AGENT

(30) Priorité: 08.03.2004 FR 0450461
(43) Date de publication de la demande: 22.11.2006
(73) Titulaire: Laboratoires Clarins, 92200 Neuilly sur Seine (FR)
(72) Inventeur: COURTIN, Olivier, F-92100 Boulogne-Billancourt (FR)
(74) Mandataire: Novagraaf IP
(86) Numéro de dépôt international: PCT/FR2005/000554
(87) Numéro de publication internationale: WO 2005/087189

(56) Documents cités:
- EP-A- 1 449 517
- WO-A-00/62789
- WO-A-20/05041916
- FR-A- 2 813 885
- JP-A- 10 226 618
- JP-A- 2000 143 460
- JP-A- 2003 252 745
- US-A1- 2002 159 971
- US-B1- 6 673 623
- BOULOUMIE A ET AL: "ADIPOCYTE PRODUCES MATRIX METALLOPROTEINASES 2 AND 9 INVOLVEMENT IN ADIPOSE DIFFERENTIATION" DIABETES, NEW YORK, NY, US, vol. 50, no. 9, septembre 2001 (2001-09), pages 2080-2086, XP008008912 ISSN: 0012-1797
- DATABASE WPI Section Ch, Week 198240 Derwent Publications Ltd., London, GB; Class B05, AN 1982-010850 XP002303421 & RO 79 244 A (INTR TERAPIA MEDICA) 30 juin 1982 (1982-06-30)
- DATABASE BIOSIS [Online] BIOSCIENCES INFORMATION SERVICE, PHILADELPHIA, PA, US; janvier 2002 (2002-01), TOSETTI FRANCESCA ET AL: "'Angioprevention': Angiogenesis is a common and key target for cancer chemopreventive agents" XP002333824 Database accession no. PREV200200090623 & FASEB JOURNAL, vol. 16, no. 1, janvier 2002 (2002-01), pages 2-14, ISSN: 0892-6638

## Description

La présente invention concerne le domaine des cosmétiques et plus particulièrement le domaine des amincissants. La présente invention concerne également une composition cosmétique amincissante comprenant en tant qu'agent actif un extrait de Baccharis genistelloides comprenant un inhibiteur de métalloprotéinases matricielles (MMP) 2 et/ou 9. La présente invention concerne ainsi des compositions utiles pour réguler la différentiation adipocytaire et réduire ainsi la lipogenèse.

Le tissu adipeux (ou hypoderme) est rattaché à la partie inférieure du derme par des expansions de fibres de collagène. Son épaisseur est variable, mince sur le front, il se développe largement sur des zones particulières (cuisses, abdomen). La localisation anatomique du tissu adipeux est un véritable caractère sexuel secondaire. Chez l'homme, il est prépondérant au-dessus de la ceinture, au niveau de l'abdomen et des épaules, alors que, chez la femme, il se concentre au-dessous de la ceinture, dans la partie basse de l'abdomen et au niveau des hanches, fesses et cuisses. Cette localisation liée au sexe est fortement soulignée en cas d'obésité dont on distingue deux formes, la forme androïde et la forme gynoïde.
Le tissu adipeux est constitué :
- de cellules spécifiques, les adipocytes qui ont la possibilité de stocker des graisses (lipogenèse) mais également de les mobiliser (lipolyse),
- d'une matrice extracellulaire,
- d'un réseau capillaire dense.
   Le développement du tissu adipeux se caractérise par une modification de l'ensemble des composants du tissu hypodermique. Ces perturbations ont également des répercussions au niveau des couches plus superficielles. Au niveau du derme, on observe une augmentation de l'épaisseur, probablement due à un engorgement liquidien (rétention d'eau) et à une infiltration de lobules adipeux déformant la jonction derme/hypoderme ; en surface la peau prend un aspect capitonné c'est la fameuse « peau d'orange ».

Le développement du tissu adipeux fait intervenir différents mécanismes :
- la réorganisation de la matrice extracellulaire,
- la différenciation des pré-adipocytes en adipocytes,
- l'augmentation du volume des adipocytes.

La matrice extracellulaire structure l'organisation de l'hypoderme, elle lui confère une certaine tenue. Elle est constituée majoritairement de collagène, élastine, protéoglycanes, fibronectine et autres protéines glycosylées. Ces macromolécules sont organisées en réseau fibrillaire complexe. Les mécanismes de dégradation qui transforment ce réseau se produisent grâce à l'action d'un ou de plusieurs membres de la famille des métalloprotéinases. A ce jour, une vingtaine de métalloprotéinases ont été identifiées. Au niveau de l'hypoderme, on retrouve les métalloprotéinases 2 et 9 (deux gélatinases) sécrétées par les adipocytes (A. Bouloumié, C. Sengenès, G. Portolan, J. Galitzky et M. Lafontan, Diabetes, 2001, vol. 50, pages 2080-2086). Les métalloprotéinases 2 et 9 dégradent la matrice extracellulaire environnante, contribuant ainsi à la dissolution de son architecture. La matrice extracellulaire ainsi déstructurée, laisse la place aux adipocytes qui réorganisent le tissu, occupent l'espace et favorisent l'apparition d'un nouveau réseau capillaire nécessaire à leur développement.
Il est maintenant bien établi que le nombre d'adipocytes peut évoluer au cours de la vie. Au niveau du tissu adipeux, la présence de cellules « précurseurs » appelées préadipocytes a été mise en évidence. Ces cellules ont la possibilité sous l'effet de stimuli (insuline et glucose, chémokines sécrétées par les adipocytes matures) de se transformer en adipocytes (cellules capables de stocker les graisses) : c'est la différenciation adipocytaire. L'augmentation du nombre d'adipocytes (ou hyperplasie du tissu) est donc un élément important dans le développement du tissu graisseux. La réorganisation de la matrice extracellulaire et la différenciation adipocytaire sont intimement liées : l'adjonction de molécules inhibitrices des métalloprotéinases à des cellules préadipocytaires permet d'inhiber leur différenciation et donc de limiter le développement du tissu adipeux. Il s'agit d'une nouvelle approche thérapeutique dans la prise en charge de la cellulite qui agit en limitant le développement du tissu adipeux et en protégeant le tissu conjonctif de l'hypoderme.

Les adipocytes ont également la faculté d'accroître leur propre capacité de stockage en augmentant la quantité des graisses stockées. La captation des acides gras par les adipocytes est réalisée grâce à une enzyme sécrétée par les adipocytes : la lipoprotéine lipase. La lipoprotéine lipase hydrolyse les triglycérides véhiculés par les lipoprotéines sanguines. Dans l'adipocyte, les acides gras sont estérifiés grâce à un métabolite du glucose et stockés essentiellement sous forme de triglycérides. Cette étape nécessite la présence d'une enzyme la glycérol 3 phosphate déshydrogénase (G3PDH).

A contrario, les adipocytes sont aussi capables de mobiliser les graisses stockées, c'est la lipolyse. La lipolyse est sous la commande d'une hormone la lipase sensible aux hormones qui scinde les triglycérides en acides gras et glycérol et permet leur élimination de la cellule. La lipase sensible aux hormones étant sous la dépendance des taux d'AMP_{c} et de GMP_{c} intracellulaires, il convient donc d'augmenter le taux d'AMP_{c} et/ou de GMP_{c} pour favoriser la lipolyse.

Il a été proposé dans l'art antérieur de nombreuses compositions cosmétiques amincissantes favorisant la lipolyse et/ou inhibant la lipogenèse. On peut citer, plus particulièrement, les compositions cosmétiques amincissantes décrites dans le brevet français de la Demanderesse publié sous le No. 2 729 856. Ce brevet propose des compositions cosmétiques amincissantes capables par le biais de substances organiques solubles, ou enzymes, d'affiner et de préparer la peau à recevoir des principes actifs dont les effets sont potentialisés pour favoriser l'élimination des lipides et prévenir le stockage de ces lipides.
Les travaux de la Demanderesse ont permis d'identifier la présence d'inhibiteurs de métalloprotéinases 2 et/ou 9 dans des extraits végétaux de Baccharis genistelloides et envisagent donc des compositions amincissantes contenant lesdits extraits.
La présente invention concerne donc une composition cosmétique amincissante caractérisée en ce qu'elle comprend en tant qu'agent actif, au moins un inhibiteur de métalloprotéinases 2 et/ou 9 sous la forme d'un extrait végétal de Baccharis genistelloides contenant cet inhibiteur, en tant que protecteur de la matrice extracellulaire et destiné à prévenir la différenciation adipocytaire.

La composition selon l'invention peut contenir un ou plusieurs extraits végétaux comprenant un inhibiteur de métalloprotéinase 2 et/ou 9. La composition selon l'invention contient un extrait de *Baccharis genistelloides.* Cette plante aromatique d'Amazonie appartient à la famille des astéracées.

De façon avantageuse, l'extrait utilisable dans le cadre de la présente invention est issu de la partie aérienne de *Baccharis genistelloides* et, de préférence, il s'agit d'un extrait hydro-glycolique. Après solubilisation de *Baccharis genistelloides* dans un mélange eau/butylène glycol (50/50), on sépare les phases soluble et insoluble par filtration. Puis on réalise une filtration stérilisante de la phase soluble pour obtenir l'extrait final de *Baccharis genistelloides* qui se présente sous la forme d'un liquide limpide de couleur ambré et d'odeur caractéristique. Il présente les caractéristiques analytiques suivantes
- pH : 5 à 6
- matières sèches : 13 à 22 g/l
- polyphénols totaux (exprimés en acide gallique) : 0,7 à 1,4.

Outre l'extrait de plante contenant au moins un inhibiteur de métalloprotéinases 2 et/ou 9, la composition de l'invention peut comprendre au moins un agent actif utile pour inhiber la lipogenèse ou favoriser la lipolyse ou encore pour affiner, raffermir, lifter la surface cutanée ou l'hydrater. Cet actif est, de préférence, la caféine qui présente une action lipolytique. De plus, la composition de l'invention peut comprendre au moins un agent actif choisi parmi les composés et extraits suivants :
- un extrait de fragon ou petit houx ou encore Ruscus, qui est riche en matières minérales telles que calcium et potassium, tout comme en saponosides et sapogénines. La présence de saponosides confère à cet extrait des propriétés :
   - anti-inflammatoire, calmante et apaisante,
   - éclaircissante, efficace pour la protection contre les rougeurs,
   - favorisant la microcirculation et donc améliorant la tonicité des capillaires,
   - décongestionnante cutanée.
- un ou plusieurs dérivés de silicium, comme le SILANOL, qui présente les propriétés suivantes :
   - hydratante permettant de régulariser la concentration hydrique
   - de prévention et de régénération, notamment une activité anti-radicalaire qui permet une réorganisation des lipides membranaires en rendant la membrane plus résistante à l'attaque des radicaux libres,
   - anti-inflammatoire, qui diminue la vasodilatation et annule l'action de l'acide arachidonique.
- un extrait de marron d'Inde, qui présente des propriétés :
   - vitaminiques P, qui permettent d'augmenter la résistance et de diminuer la perméabilité des capillaires sanguins (vasoprotecteur),
   - décongestionnantes et adoucissantes sur l'inflammation, car les dérivés saponosiques du marron d'Inde permettent de traiter l'effet "peau d'orange".
- un extrait de Ginkgo Biloba, dont les feuilles sèches sont utilisées en médecine comme tonique veineux du fait de leur teneur en flavonoïdes.
- un extrait de vigne rouge. Les feuilles de vigne rouge sont riches en flavonoïdes et permettent de renforcer les parois des vaisseaux et le tonus veineux. Un tel extrait agit donc sur la microcirculation.

La proportion de l'extrait de plante contenant au moins un inhibiteur de métalloprotéinases 2 et/ou 9 dans la composition selon l'invention est comprise entre 0,1 à 10%, de préférence de l'ordre de 0,1 à 5 % (poids/poids).

La composition selon l'invention peut encore comprendre un ou plusieurs agents de formulation ou additifs d'usage connu et classique dans les compositions cosmétiques et dermatologiques telles que, à titre d'exemple et de façon non limitative, des adoucissants, des colorants, des actifs filmogènes, des tensio-actifs, des conservateurs, des émulsionnants, des huiles, des glycols, des vitamines, etc... Grâce à ces connaissances en matière de cosmétiques, l'homme du métier saura quels agents de formulation ajouter à la composition de l'invention et en quelles quantités en fonction des propriétés recherchées.

La composition de l'invention peut se présenter sous toute forme connue de l'homme du métier dans le domaine de la cosmétique, comme par exemple, une crème, un lait, une lotion, un gel, un masque, etc..., sans aucune restriction galénique particulière autre que celles pour l'application sur la peau.

La présente invention concerne l'utilisation d'un extrait de Baccharis genistelloides comprenant un inhibiteur de métalloprotéinases 2 et/ou 9 tel que défini précédemment comme agent actif amincissant. En outre, la présente invention concerne l'utilisation cosmétique d'un inhibiteur de métalloprotéinases 2 et/ou 9 tel que défini précédemment comme agent prévenant la différenciation adipocytaire, pour la préparation d'une composition amincissante.

Les exemples ci-après sont donnés à titre illustratif et ne sauraient être interprétés comme limitant la portée de l'invention. Ils concernent, d'une part, la mise en évidence du rôle d'un extrait de *Baccharis genistelloides* sur l'inhibition de la différenciation adipocytaire et sur l'inhibition de la sécrétion des métalloprotéinases 2 et 9 et de leur activité, et, d'autre part, des exemples de compositions objet de la présente invention.

Les exemples font référence aux figures suivantes dans lesquelles :

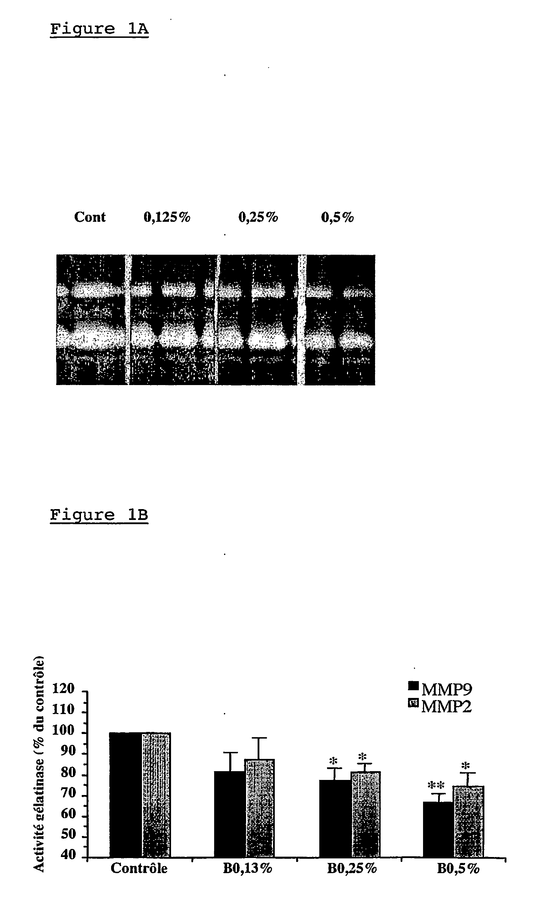

- la figure 1 représente les effets de l'extrait de *Baccharis genistelloides* sur l'activité des métalloprotéinases adipocytaires MMP-2 et MMP-9 (n=3). Les effets du produit ont été testés sur l'activité des métalloprotéinases MMP-2 et MMP-9 présentes dans des surnageants de culture de préadipocytes différenciés 5 jours. (A) Photographie de gels représentatifs. (B) Quantification des zymographies. * P≤0,05 ou ** P≤0,01 traité *vs* contrôle.

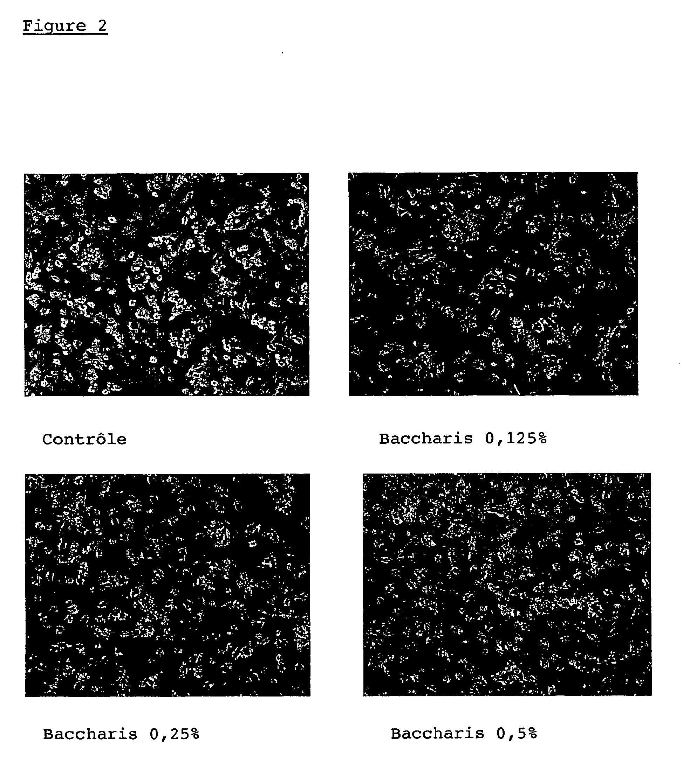

- la figure 2 représente les préadipocytes différenciés 10 jours en présence de l'extrait de *Baccharis genistelloides* (0,125%, 0,25% ou 0,5%).

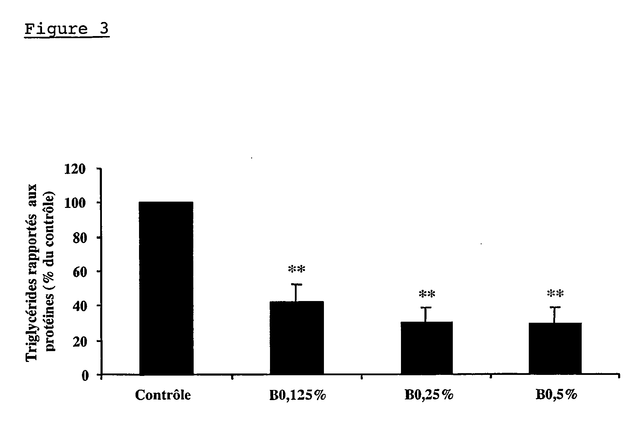

- la figure 3 représente les effets d'un extrait de *Baccharis genistelloides* sur les taux de triglycérides intracellulaires dans les préadipocytes (n=4). Les préadipocytes différenciés 10 jours en présence de l'extrait de *Baccharis genistelloides* (0,125%, 0,25% ou 0,5%). Les taux de triglycérides ont été rapportés aux taux de protéines et sont exprimés en pourcentage du contrôle. * P≤0,05, ** P≤0,01 traité *vs* contrôle.

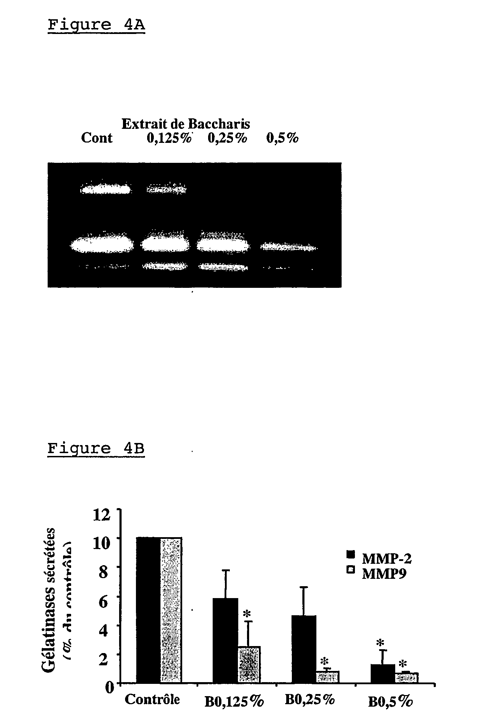

- la figure 4 représente les effets d'un extrait de *Baccharis genistelloides* sur la sécrétion des gélatinases MMP-2 et MMP-9 par les préadipocytes (n=4). Les activités gélatinases ont été mesurées dans les surnageants de culture par zymographie à la gélatine et sont exprimées en % du contrôle. (A) Photographie ** P≤0,01 traité *vs* contrôle. (B) Quantification des zymographies.

### I. ACTIVITE DE L'EXTRAIT DE BACCHARIS GENISTELLOIDES.

### A. Matériel et Méthodes.

### 1. Culture et traitements des préadipocytes humains.

Le tissu adipeux sous-cutané provient de personnes en surcharge pondérale ou modérément obèses, ayant subi une chirurgie plastique (dermo-lipectomie abdominale). Après digestion à la collagénase sous agitation, les cellules de la fraction stroma-vasculaire sont ensemencées dans du milieu Eagle modifié par Dulbecco (DMEM)/F12 supplémenté avec 10% de sérum de veau foetal (SVF) et un mélange d'antibiotiques (biotine 33µM, D-panthoténate 17µM, gentamycine 50µg/ml) et de nouveau filtré (filtre 70µm). Les cellules sont incubées dans une atmosphère de 95% d'air - 5% de CO₂ à 37°C. Après 24h, le milieu est remplacé par du DMEM/F12 additionné d'antibiotiques et d'hormones : insuline 22µM, transferrine 10µg/ml, cortisol 100µM, T3 2µM ainsi que de la ciglitizone 1mg/ml (agoniste PPAR) pendant 72h. Après ces 72h, le milieu est remplacé par le même milieu sans ciglitizone, avec ou sans l'extrait de *Baccharis genistelloides* (B 0,125%, B 0,25%, B 0,5%).

Ce jour est considéré comme J0. Ce milieu différenciant est renouvelé tous les deux jours. Après 10 jours de différenciation, les surnageants sont collectés et stockés à -20°C jusqu'aux dosages et analyses par zymographie. Les cellules sont congelées à -20°C après un lavage avec du PBS.

### 2. Dosages des protéines et des triglycérides.

Les cellules sont recueillies dans 100µl de PBS, lysées par sonication, et les quantités de protéines et de triglycérides intracellulaires sont dosées à l'aide des kits DC Bio-Rad Protein Assay et GPO-Trinder de Bio-Mérieux.

### 3. Zymographie à la gélatine.

Les protéines ayant une activité gélatinase (MMP-2 et MMP-9) sont identifiées par électrophorèse sur des gels de SDS-polyacrylamide (SDS-PAGE) contenant un substrat des MMPs : la gélatine (1 mg/ml) (ICN Biomedicals).
a- Afin de déterminer l'effet de l'extrait de *Baccharis genistelloides* sur la sécrétion des MMPs par les cellules, le milieu de culture des cellules traitées avec les agents (20 µl + 10µl de bleu de dépôt non dénaturant) est déposé directement sur les gels. Après électrophorèse, les protéines sont renaturées en incubant les gels avec 2,5 % de Triton X-100 (incubation : deux fois 15 min). Les gels sont ensuite incubés durant 16h à 37°C dans 50 mmol/l Tris-HCl (pH 8,5), 0,02 % NaN₃ et 5 mmol/l CaCl₂. En fin d'incubation, les gels sont colorés au Bleu de Coomassie et l'activité gélatinase est visualisée par des régions de lyse sur le gel coloré.
b- Afin de déterminer l'effet de l'extrait de *Baccharis genistelloides* sur l'activité des MMPs, le milieu de culture de cellules contrôles différenciées pendant 5 jours (20 µl + 10 µl de bleu de dépôt non dénaturant (1 mol/l Tris HCl, pH 6,8, glycérine, Sodium Dodécyl Sulfate 20 %, β-mercaptoéthanol, Bleu de Bromophénol 0,05 %)) est déposé directement dans les gels. Après électrophorèse, les protéines sont renaturées en incubant les gels avec 2,5% de Triton X-100 (incubation : deux fois 15 min). Les gels sont ensuite incubés durant 16h à 37°C dans 50 mmol/l Tris-HCl (pH 8,5), 0,02% NaN₃ et 5 mmol/l CaCl₂ en absence ou en présence de l'extrait de *Baccharis genistelloides* (B 0,125%, B 0,25%, B 0,5%). En fin d'incubation, les gels sont colorés au Bleu de Coomassie (50% méthanol, 10% acide acétique, 0,1% de Coomassie Brillant Blue, 40% eau distillée) et l'activité gélatinase est visualisée par des régions de lyse sur le gel coloré.

### 4. Analyses statistiques.

Les valeurs données sont exprimées en pourcentage du contrôle ± écart standard à la moyenne (esm) pour (n) expériences indépendantes. Les analyses statistiques sont réalisées par analyses de variances un facteur (ANOVA) suivies d'un test *post hoc* de Dunnett. Les valeurs sont considérées significatives quand p<0,05.

### B. Résultats.

### 1. Effet de l'extrait de Baccharis genistelloides sur l'activité des MMPs.

Nous avons étudié l'effet de l'extrait de *Baccharis genistelloides* sur l'activité des MMPs sécrétées par des préadipocytes humains différenciés 5 jours (MMP-2 et MMP-9). La figure 1 montre que l'extrait de *Baccharis genistelloides* inhibe de manière dépendante de la concentration l'activité de MMP-2 et MMP-9.

### 2. Effet de l'extrait de Baccharis genistelloides sur la différenciation des préadipocytes humains en culture primaire.

Nous avons traité avec l'extrait de *Baccharis genistelloides* des préadipocytes humains issus de 2 dermo-lipectomies abdominales (DLA) différentes.

La figure 2 montre les effets du produit sur la morphologie des préadipocytes humains en culture primaire après 10 jours de traitement. On observe une diminution de la quantité de gouttelettes lipidiques dans les cellules traitées par rapport aux cellules contrôles.

Nous avons pu observer une diminution significative du contenu intracellulaire en triglycérides (rapporté aux mg de protéines) dans tous les cas de figure (Fig. 3).

Nous avons enfin étudié l'effet de l'extrait de *Baccharis genistelloides* sur la sécrétion de MMP-2 et MMP-9 par les préadipocytes, et nous avons montré (Fig. 4) une diminution significative de la sécrétion des deux protéases avec le traitement.

### II. EXEMPLES DE COMPOSITIONS OBJET DE L'INVENTION

### A. GEL CREME AMINCISSANT.

| | | |
|---|---|---|
| EAU DEMINERALISEE | Q.S.P | 100 |
| PEMULEN TRI | | 0,5 |
| GLYCERINE | | 5,0 |
| CAFEINE ANHYDRE | | 2,0 |
| POB BUTYL | | 0,05 |
| HUILE DE SILICONE | | 7,5 |
| C12 - C15 ALKYL BENZOATE | | 1,5 |
| EXTRAIT DE BACCHARIS GENISTELLOIDES | | 1,0 |
| DERIVE DE SILICIUM | | 3,0 |
| EXTRAIT DE GERANIUM ROBERTIANUM | | 0,5 |
| TEA | | 0,5 |
| ALCOOL DENATURE A 96° | | 15,0 |
| MENTHOL | | 0,35 |
| PARFUM | | 0,6 |
| COLORANTS A 1 % | | 0,12 |

### B. EMULSION AMINCISSANTE.

| | |
|---|---|
| HUILE VEGETALE | 1,0 |
| HUILE DE SILICONE | 15,0 |
| ISONONANOATE D'ISONONYLE | 10,0 |
| ALCOOL CETYLIQUE | 1,0 |
| CETEARYL GLUCOSIDE | 5,0 |
| GLYCERINE | 3,0 |
| SEPIGEL 305 | 0,2 |
| CAFEINE ANHYDRE | 2,0 |
| EXTRAIT DE BACCHARIS GENISTELLOIDES | 1,0 |
| EXTRAIT DE GINGKO BILOBA | 1,0 |
| EXTRAIT DE VIGNE ROUGE | 1,0 |
| MENTHOL | 0,25 |
| PHENONIP | 0,8 |
| SORBATE DE POTASSIUM | 0,1 |
| PARFUM | 0,5 |

### C. GEL AMINCISSANT.

| | | |
|---|---|---|
| EAU DEMINERALISEE | Q.S.P | 100 |
| PEMULEN TR 1 | | 0,2 |
| CARBOMER | | 0,4 |
| CAFEINE ANHYDRE | | 2,0 |
| EXTRAIT DE PETIT HOUX | | 1,0 |
| EXTRAIT DE MARRON D'INDE | | 1,0 |
| EXTRAIT DE BACCHARIS GENISTELLOIDES | | 1,0 |
| TEA | | 0,65 |
| ALCOOL DENATURE A 96° | | 15,0 |
| MENTHOL | | 0,4 |
| PARFUM | | 0,4 |

## Revendications

1. Utilisation cosmétique d'un extrait de Baccharis genistelloides comprenant au moins un inhibiteur de métalloprotéinases 2 et/ou 9 comme agent actif amincissant.

2. Utilisation d'un extrait de plante selon la revendication 1, dans laquelle ledit extrait de plante est un extrait hydro-glycolique de la plante.

3. Utilisation d'un extrait de plante selon l'une quelconque des revendications précédentes, dans laquelle ladite composition cosmétique amincissante comprend en outre au moins un agent actif utile pour inhiber la lipogenèse ou favoriser la lipolyse ou encore pour affiner, raffermir, lifter la surface cutanée ou l'hydrater.

4. Utilisation d'un extrait de plante selon la revendication 3, dans laquelle ladite composition cosmétique amincissante comprend de la caféine.

5. Utilisation d'un extrait de plante selon l'une quelconque des revendications 3 ou 4, dans laquelle ladite composition cosmétique amincissante comprend au moins un agent actif choisi parmi un extrait de petit houx, un dérivé de silicium, un extrait de marron d'Inde, un extrait de Ginkgo biloba ou un extrait de vigne rouge.

6. Utilisation d'un extrait de plante selon l'une quelconque des revendications précédentes, dans laquelle la proportion d'extrait de plante contenant au moins un inhibiteur de métalloprotéinases 2 et/ou 9 dans ladite composition cosmétique amincissante est comprise entre 0,1 à 10%, et de préférence de 0,1 à 5%.

7. Procédé de soin cosmétique amincissant consistant à appliquer sur la peau un extrait de Baccharis genistelloides comprenant au moins un inhibiteur de métalloprotéinases 2 et/ou 9 ou une composition comprenant cet extrait de plante comme agent actif amincissant.

8. Procédé de soin cosmétique amincissant selon la revendication 7, dans lequel ledit extrait de plante est un extrait hydro-glycolique de la plante.

9. Procédé de soin cosmétique amincissant selon la revendication 7 ou 8, dans lequel ladite composition comprend en outre au moins un agent actif utile pour inhiber la lipogenèse ou favoriser la lipolyse ou encore pour affiner, raffermir, lifter la surface cutanée ou l'hydrater.

10. Procédé de soin cosmétique amincissant selon la revendication 9, dans lequel ladite composition comprend de la caféine.

11. Procédé de soin cosmétique amincissant selon la revendication 9 ou 10, dans lequel ladite composition comprend au moins un agent actif choisi parmi un extrait de petit houx, un dérivé de silicium, un extrait de marron d'Inde, un extrait de Ginkgo biloba ou un extrait de vigne rouge.

12. Procédé de soin cosmétique amincissant selon l'une quelconque des revendications 7 à 11, dans lequel la proportion d'extrait de plante contenant au moins un inhibiteur de métalloprotéinases 2 et/ou 9 dans ladite composition est comprise entre 0,1 à 10%, et de préférence de 0,1 à 5%.

## Claims

1. Cosmetic use of a Baccharis genistelloides extract comprising at least one metalloproteinase inhibitor 2 and/or 9 as an active slimming agent.

2. Use of a plant extract according to claim 1, wherein said plant extract is a hydro-glycolic extract of the plant.

3. Use of a plant extract according to any of the above claims, wherein said cosmetic slimming formulation further comprises at least one active agent useful for inhibiting lipogenesis or promoting lipolysis or for slimming, firming, lifting or hydrating the skin surface.

4. Use of a plant extract according to claim 3, wherein said cosmetic slimming formulation comprises caffeine.

5. Use of a plant extract according to any of claims 3 or 4, wherein said cosmetic slimming formulation comprises at least one active agent selected from a butcher's broom extract, a silicon derivative, a horse chestnut extract, a Ginkgo biloba extract or a red vine extract.

6. Use of a plant extract according to any of the above claims, wherein the proportion of plant extract containing at least one metalloproteinase inhibitor 2 and/or 9 in said cosmetic slimming formulation is in the range between 0.1 and 10%, and preferentially from 0.1 to 5%.

7. Cosmetic slimming treatment process consisting of applying, on the skin, a Baccharis genistelloides extract comprising at least one metalloproteinase inhibitor 2 and/or 9 or a formulation comprising said plant extract as an active slimming agent.

8. Cosmetic slimming treatment process according to claim 7, wherein said plant extract is a hydro-glycolic extract of the plant.

9. Cosmetic slimming treatment process according to claim 7 or 8 , wherein said cosmetic slimming formulation further comprises at least one active agent useful for inhibiting lipogenesis or promoting lipolysis or for slimming, firming, lifting or hydrating the skin surface.

10. Cosmetic slimming treatment process according to claim 9, wherein said formulation comprises caffeine.

11. Cosmetic slimming treatment process according to claim 9 or 10, wherein said formulation comprises at least one active agent selected from a butcher's broom extract, a silicon derivative, a horse chestnut extract, a Ginkgo biloba extract or a red vine extract.

12. Cosmetic slimming treatment process according to any of claims 7 to 11, wherein the proportion of plant extract containing at least one metalloproteinase inhibitor 2 and/or 9 in said cosmetic slimming formulation is in the range between 0.1 and 10%, and preferentially from 0.1 to 5%.

## Patentansprüche

1. Kosmetische Verwendung eines Extrakts aus Baccharis genistelloides, umfassend mindestens einen Inhibitor der Metalloprotease-2 und/oder -9 als Schlankmacher-Wirkstoff.

2. Verwendung eines Pflanzenextrakts nach Anspruch 1, wobei der Pflanzenextrakt ein hydroglykolischer Extrakt der Pflanze ist.

3. Verwendung eines Pflanzenextrakts nach einem der vorhergehenden Ansprüche, wobei die kosmetische Schlankmacher-Verbindung außerdem mindestens einen Wirkstoff umfasst, der dazu dient, die Lipogenese zu hemmen oder die Lipolyse zu fördern oder auch die Hautoberfläche zu verfeinern, zu straffen, zu liften oder sie zu hydratisieren.

4. Verwendung eines Pflanzenextrakts nach Anspruch 3, wobei die kosmetische Schlankmacher-Verbindung Koffein enthält.

5. Verwendung eines Pflanzenextrakts nach einem der Ansprüche 3 oder 4, wobei die kosmetische Schlankmacher-Verbindung mindestens einen Wirkstoff, ausgewählt unter einem Extrakt des Mäusedorns, einem Siliziumderivat, einem Extrakt der Rosskastanie, einem Extrakt aus Ginkgo biloba oder einen Extrakt der roten Weinrebe, enthält.

6. Verwendung eines Pflanzenextrakts nach einem der vorhergehenden Ansprüche, wobei der Pflanzenextrakt-Anteil, umfassend mindestens einen Inhibitor der Metalloprotease-2 und/oder -9, in der kosmetischen Schlankmacher-Verbindung zwischen 0,1 und 10% und vorzugsweise zwischen 0,1 und 5% liegt.

7. Kosmetisches Schlankmacher-Pflegeverfahren, umfassend die Anwendung eines Extraktes aus Baccharis genistelloides auf die Haut, enthaltend mindestens einen Inhibitor der Metalloprotease-2 und/oder -9, oder eine Verbindung umfassend diesen Pflanzenextrakt als Schlankmacher-Wirkstoff.

8. Kosmetisches Schlankmacher-Pflegeverfahren nach Anspruch 7, wobei der Pflanzenextrakt ein hydroglykolischer Extrakt der Pflanze ist.

9. Kosmetisches Schlankmacher-Pflegeverfahren nach Anspruch 7 oder 8, wobei die Verbindung außerdem mindestens einen Wirkstoff umfasst, der dazu dient, die Lipogenese zu hemmen oder die Lipolyse zu fördern und auch die Hautoberfläche zu verfeinern, zu straffen, zu liften oder sie zu hydratisieren.

10. Kosmetisches Schlankmacher-Pflegeverfahren nach Anspruch 9, wobei die Verbindung Koffein enthält.

11. Kosmetisches Schlankmacher-Pflegeverfahren nach Anspruch 9 oder 10, wobei die Verbindung mindestens einen Wirkstoff, ausgewählt unter einem Extrakt des Mäusedorns, einem Siliziumderivat, einem Extrakt der Rosskastanie, einem Extrakt aus Ginkgo biloba oder einen Extrakt der roten Weinrebe, enthält.

12. Kosmetisches Schlankmacher-Pflegeverfahren nach einem der Ansprüche 7 bis 11, wobei der Pflanzenextrakt-Anteil, umfassend mindestens einen Inhibitor der Metalloprotease 2 und/oder -9, in der Verbindung zwischen 0,1 und 10% und vorzugsweise zwischen 0,1 und 5% liegt.
